# EUROPEAN PATENT SPECIFICATION

(11) **EP 3 661 485 B1**
(45) Date of publication and mention of the grant of the patent: **07.10.2026**
(21) Application number: 18841065.8
(22) Date of filing: 06.08.2018
(51) Int. Cl.: A61K 9/00, A61M 37/00, A61M 5/158, C12N 13/00, C12N 15/113, C12N 15/88, C12M 1/42

(54) **METHOD FOR PRODUCING THERAPEUTIC EXOSOMES FROM NANOELECTROPORATION AND OTHER NON-ENDOCYTIC CELL TRANSFECTION**
VERFAHREN ZUR HERSTELLUNG VON THERAPEUTISCHEN EXOSOMEN AUS NANOELEKTROPORATION UND ANDEREN NICHT-ENDOZYTISCHEN ZELLTRANSFEKTIONEN
PROCÉDÉ DE PRODUCTION D'EXOSOMES THÉRAPEUTIQUES À PARTIR DE NANOÉLECTROPORATION ET AUTRE TRANSFECTION DE CELLULE NON ENDOCYTIQUE

(30) Priority: 04.08.2017 US 201762541157 P
(43) Date of publication of application: 10.06.2020
(60) Divisional application: 26198288.8
(73) Proprietor: Ohio State Innovation Foundation, Columbus, OH 43201 (US)
(72) Inventor: LEE, Ly, James, Columbus OH 43220 (US); SHI, Junfeng, Columbus OH 43214 (US); YANG, Zhaogang, Columbus OH 43220 (US)
(74) Representative: Wynne-Jones IP Limited
(86) International application number: PCT/US2018/045333
(87) International publication number: WO 2019/028450

(56) References cited:
- WO-A1-2017/054086
- US-A1- 2014 256 047
- SHI JUNFENG: "Development of Nanoelectroporation-based Biochips for Living Cell Interrogation and Extracellular", THESIS, 10 May 2017 (2017-05-10), Ohio USA, pages 130 - 136, XP055790849, Retrieved from the Internet <URL:http://rave.ohiolink.edu/etdc/view?acc_num=osu1503059915552435> [retrieved on 20210329]
- CHANG LINGQIAN ET AL: "3D nanochannel electroporation for high-throughput cell transfection with high uniformity and dosage control", NANOSCALE, vol. 8, no. 1, 1 January 2016 (2016-01-01), United Kingdom, pages 243 - 252, XP055790463, ISSN: 2040-3364, DOI: 10.1039/C5NR03187G
- CHANG LINGQIAN ET AL: "3D nanochannel electroporation for high-throughput cell transfection with high uniformity and dosage control", NANOSCALE ENGLAND, vol. 8, no. 1, 7 January 2016 (2016-01-07), pages 243 - 252, XP055510019
- MIZRAK ET AL.: "Genetically engineered microvesicles carrying suicide mRNA/protein inhibit schwannoma tumor growth", MOLECULAR THERAPY, vol. 21, no. 1, 1 January 2013 (2013-01-01), pages 101 - 108, XP055513776

## Description

### Technical Field

The present invention relates to methods for producing therapeutic extracellular vesicles (EVs), exosomes in particular, that contain functional messenger RNAs (mRNAs), microRNAs (miRs), short hairpin RNAs (shRNAs), proteins, and other biomolecules by non-endocytic delivery of DNA plasmids and other vectors into donor cells in a way that the strong stimulation caused by delivery triggers donor cells to generate a large number of vesicles within the cell while the non-endocytic delivery of DNA plasmids/vectors leads to fast transcription of RNAs and translation of proteins within cytoplasm, allowing those functional biomolecules to be encapsulated in the vesicles endogenously before they are secreted out from donor cells as EVs.

### Background

Extracellular vesicles (EVs), including exosomes, microvesicles and other vesicles, are secreted by numerous cell types. In the human body, there are >10E12 EVs in 1 mL blood and they also exist in various body fluids. Exosomes are nano-vesicles (40-150 nm), while microvesicles have sizes varied from <100 nm to >1 micron. They contain both coding and non-coding RNAs and their fragments, DNA fragments, proteins, and other cell related biomolecules. EVs and their biomolecule contents have been proposed as biomarkers for disease diagnosis. In addition, they play major roles in cell-cell communications in tumor microenvironment and circulation.

EVs loaded with functional RNAs and proteins have also been suggested as drugs and drug carriers for therapeutic applications. To deliver specific nucleic acids and/or proteins to target tissues or cell types *in vitro* and *in vivo* requires methods that can produce EVs with either endogenous or exogenous therapeutic cargos.

Post-insertion of exogenous small interference RNA (siRNA) and shRNA plasmids into pre-existed exosomes by conventional bulk electroporation (BEP) has been developed in recent years. Although their therapeutic functions have been successfully demonstrated in several mouse models for cancer and non-cancer diseases, this approach faces many limitations. First, post-insertion of large biomolecules such as DNA plasmids, mRNAs, and proteins into nano-sized exosomes is inefficient. Secondly, the strong electric field generated by BEP would break up many exosomes leading to a low yield of therapeutic exosomes. Furthermore, many large biomolecules such as mRNAs and proteins are difficult and expensive to be synthesized exogenously.

It would be highly desirable if new methods can be developed, that may transfect donor cells with DNA plasmids or other vectors to produce a large number of exosomes or other EVs containing therapeutic RNA and protein targets endogenously.

In a prior US patent application 14/282630, we developed a nanochannel electroporation (NEP) biochip that can deliver DNA plasmids or other charged particulates and molecules into individual cells non-endocytically with good dosage control. Herein, we demonstrate that NEP can produce a large number of therapeutic exosomes containing high copies of functional mRNA and microRNA targets, not achievable by the aforementioned post-insertion methods. In addition to NEP, other non-endocytic delivery methods such as gene gun, micro/nano-injection, etc. may also achieve a similar performance if they can provide proper cell stimulation and fast plasmid/vector delivery.

### Summary

The present invention is related to the development of new concept and methods that DNA plasmids and other vectors can be non-endocytically delivered into donor cells with strong cellular stimulation such that a large number of vesicles and transcribed RNAs as well as translated proteins are formed within the transfected cells. Cells would secret many extracellular vesicles (EVs) containing specific RNA and protein targets with therapeutic functions.

To demonstrate the aforementioned design concept, a three-dimensional (3D) NEP biochip is fabricated, that can transfect many donor cells with pre-specified DNA plasmids to secrete 10~100 fold more EVs, including exosomes, containing high copies of intact mRNA and miR targets up to many thousands fold more than those in EVs secreted from the non-transfected donor cells.

Some aspects of the invention are achieved by a method of producing a large number of therapeutic extracellular vesicles (EVs) comprising high copies of a biomolecule. Such a method comprises the steps of:
laying donor cells on a surface of a chip, the surface having a three dimensional (3D) nanochannel electroporation (NEP) biochip formed thereon;
adding a plasmid, other transfection vector, or their combinations to a buffer on the chip;
applying a pulsed electric field across the donor cells laid on top of the chip surface and plasmid/vector buffer solution below the chip surface, resulting in strongly stimulating the donor cells and delivering the plasmid/vector into the donor cells non-endocytically; and
collecting EVs secreted by the transfected donor cells.

In some of these methods, the diameter of nanochannels of the 3D NEP biochip is between 50-900 nm.

In some of these methods, the plasmid or vector transcribes messenger RNA (mRNA), microRNA (miRNA), short hairpin RNA (shRNA), or another RNA, and, optionally, lead to translation of a protein or other biomolecule in the transfected donor cells.

In some embodiments of the method, the EVs secreted by the transfected donor cells comprise the transcribed messenger RNA (mRNA), microRNA (miRNA), short hairpin RNA (shRNA), or another RNA, and, optionally, the translated proteins or other biomolecule.

In some embodiments, the method further comprises increasing the expression of heat shock proteins and other proteins that can promote vesicle formation and exocytosis in the transfected donor cells by thermal shock treatment of the donor cells, or addition of heat shock proteins.

In some embodiments, the method further comprises increasing the expression of proteins that promote exosome formation in the transfected donor cells by co-transfection of CD63, CD9 and other DNA plasmid.

In some embodiments, multiple DNA plasmids or other vectors are delivered to the transfected donor cells sequentially to promote co-localization of RNA targets or protein targets and EV secretion.

In some embodiments, exogenous biomolecules such as a DNA plasmid, other transfection vectors, RNAs, proteins/peptides or small molecule drugs are encapsulated within vesicles in the donor cells and secreted out as therapeutic EVs by sequential transfection of the donor cells by NEP. In some of these cases, in addition to NEP, one or more cell transfection methods are used to provide strong stimulation to the donor cells to facilitate EV secretion and non-endocytic plasmid/vector delivery for fast RNA transcription and protein translation to produce therapeutic EVs with similar efficacy. In further of these cases, the other cell transfection methods include, gene gun, and microinjection or nanoinjection.

In some of the embodiments, the cell transfection method comprises tethering the plasmid and/or other vector on nano- or micron-sized gold or other solid particles, and those particles are injected into the donor cells under a pneumatic force using a gene gun to cause strong stimulation of the donor cells and non-endocytic plasmis/vector delivery.

In some of the embodiments, the cell transfection method comprises tethering the the plasmid and/or other vector on a nano- or micron-sized tip array, and the donor cells are pultruded by those tips to cause strong stimulation of the donor cells and non-endocytic plasmids/vector delivery into the donor cells. In some embodiments, the plasmid or transfection vector is a DNA plasmid or DNA transfection vector, optionally wherein the EVs secreted by the transfected cells may comprise therapeutic RNA.

Also disclosed is a device for producing a large number of therapeutic extracellular vesicles (EVs) comprising high copies of a biomolecule, comprising: a chip comprising a plurality of nanochannels and a single layer of donor cells laid on a chip surface; and a buffer solution comprising a biomolecule; wherein the device is configured to apply an electric field across the plurality of nanochannels. The buffer solution may be below the chip surface. The chip may comprise a three-dimensional (3D) nanochannel electroporation (NEP) chip.

Other aspects of the disclosure comprise cells transfected by any of the foregoing methods.

### Brief Description of the Drawings

Many aspects of the disclosure can be better understood with reference to the following drawings. The components in the drawings are not necessarily to scale, emphasis instead being placed upon clearly illustrating the principles of the present disclosure. In the drawings:
**Figure 1** is a schematic of a 3D Nanochannel Electroporation (NEP) biochip for donor cell transfection;
**Figure 2** shows a comparison of BEP and NEP based cell transfection at 1 hr post-transfection using Yoyo-1 fluorescence labelled Achaete-Scute Complex Like-1 (Ascl1) DNA plasmid, a neuronal related gene;

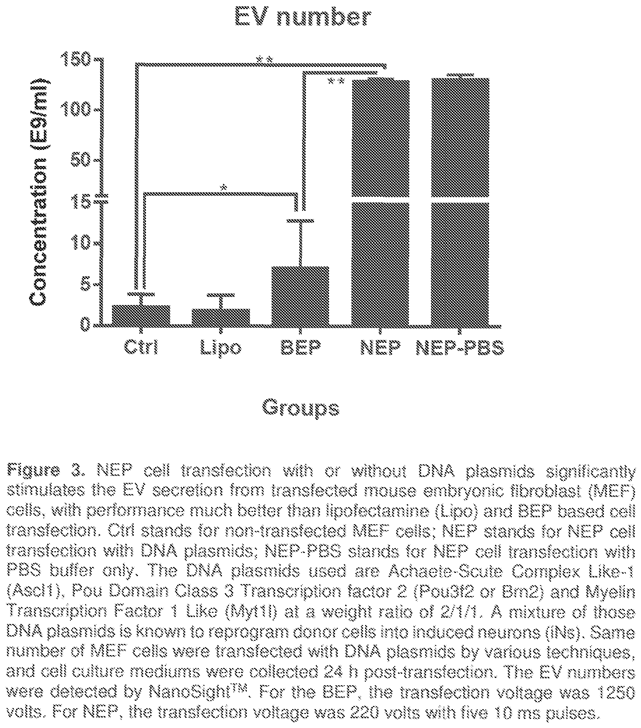

**Figure 3** shows NEP cell transfection with or without DNA plasmids significantly stimulates the EV secretion from transfected mouse embryonic fibroblast (MEF) cells, with performance much better than lipofectamine (Lipo) and BEP based cell transfection. Ctrl stands for non-transfected MEF cells; NEP stands for NEP cell transfection with DNA plasmids; NEP-PBS stands for NEP cell transfection with PBS buffer only. The DNA plasmids used are Achaete-Scute Complex Like-1 (Ascl1), Pou Domain Class 3 Transcription factor 2 (Pou3f2 or Brn2) and Myelin Transcription Factor 1 Like (Myt1l) at a weight ratio of 2/1/1. A mixture of those DNA plasmids is known to reprogram donor cells into induced neurons (iNs);
**Figure 4** shows the effect of heat shock protein 70 (HSP70) and heat shock protein 90 (HSP90) inhibitors on EV secretion from NEP transfected MEF cells. After NEP transfection, the cell culture was replaced with fresh medium containing HSP70 inhibitor (VER 155008, 50µM), HSP90 inhibitor (NVP-HSP990, 1µM), or their mixture. Medium was collected at 24 h post-transfection, and EV numbers were detected by dynamic light scattering (DLS) goniometry;
**Figure 5** shows the effect of NEP transfection of CD63 DNA plasmid on EV secretion from MEF cells. Cells were transfected with or without CD63 plasmid by NEP. The cell culture medium was collected and replaced with fresh medium every 4 hr. The EV numbers were detected by DLS goniometry;
**Figure 6** shows a size distribution measured by DLS of EVs with or without NEP harvested at 24 h post-cell transfection;

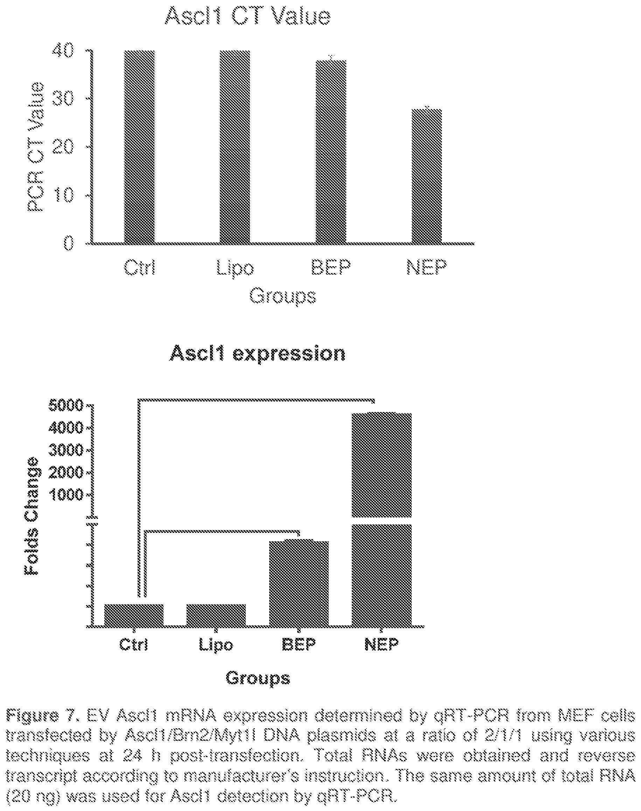

**Figure 7** demonstrates EV Ascl1 mRNA expression determined by qRT-PCR from MEF cells transfected by Ascl1/Brn2/Myt1l DNA plasmids at a ratio of 2/1/1 using various techniques at 24 h post-transfection;
**Figure 8** shows EV Brn2 mRNA expression determined by qRT-PCR from MEF cells transfected by Ascl1/Brn2/Myt1l DNA plasmids at a ratio of 2/1/1 using various techniques at 24 h post-transfection;
**Figure 9** shows EV Myt1l mRNA expression determined by qRT-PCR from MEF cells transfected by Ascl1/Brn2/Myt1l DNA plasmids at a ratio of 2/1/1 using various techniques at 24 h post-transfection;
**Figure 10** shows that only EVs obtained by NEP contain functional mRNA determined by *in vitro* translation;
**Figure 11** shows that EV-mRNAs from NEP are found in exosomes, not in microvesicles.
**Figure 12** shows that exosome-mRNAs, not microvesicle-RNAs, from NEP cell transfection can translate proteins.
**Figure 13** shows EV-mRNAs secretion profiles from NEP transfected MEF cells;
**Figure 14** depicts action potential detection by patch clamp shows that MEF cells transfected every other day with Ascl1/Brn2/Myt1l mRNA containing EVs obtained from NEP could be reprogrammed into functional induced neurons (iNs) after 24 days.
**Figure 15** shows EV miR-128 expression determined by qRT-PCR from MEF cells transfected by miR-128 DNA plasmid using various techniques at 24 h post-transfection;
**Figure 16** is a comparison of secreted EVs containing miR-128 by NEP transfection of DNA plasmid to MEF cells vs. existing EVs loaded with pre-collected miR-128 by BEP post-insertion;
**Figure 17** is a comparison of secreted EVs containing Brn2 mRNA by NEP transfection of DNA plasmid to MEF cells vs. existing EVs loaded with pre-collected Brn2 mRNA by BEP post-insertion; and

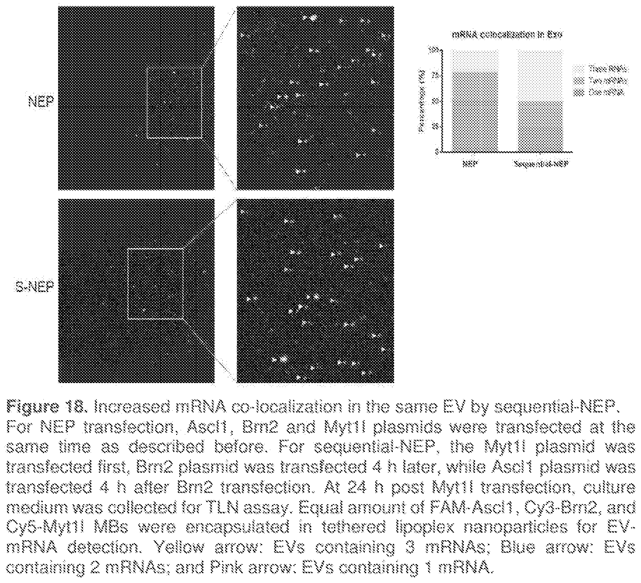

**Figure 18** shows increased mRNA co-localization in the same EV by sequential-NEP. For NEP transfection, Ascl1, Brn2 and Myt1l plasmids were transfected at the same time as described before. For sequential-NEP, the Myt1l plasmid was transfected first, Brn2 plasmid was transfected 4 h later, while Ascl1 plasmid was transfected 4 h after Brn2 transfection. At 24 h post Myt1l transfection, culture medium was collected for TLN assay. Equal amount of FAM-Ascl1, Cy3-Brn2, and Cy5-Myt1l MBs were encapsulated in tethered lipoplex nanoparticles for EV-mRNA detection. Yellow arrow: EVs containing 3 mRNAs; Blue arrow: EVs containing 2 mRNAs; and Pink arrow: EVs containing 1 mRNA.

### Detailed Description

### Example 1- 3D NEP Biochip Schematic and Comparison of EV Secretion and EV mRNA Content Using Different Transfection Methods.

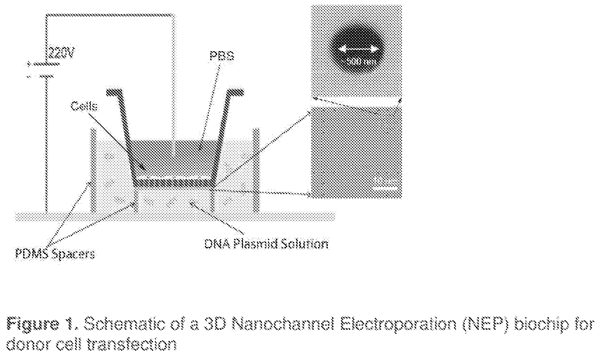

**Figure 1** shows the schematic of a 3D NEP biochip with a single layer of donor cells laid on the chip surface. After overnight cell incubation, the DNA plasmids pre-loaded in PBS buffer were injected into individual donor cells via nanochannels using a 220 volts electric field across the nanochannels. Various electroporation conditions such as voltage level, pulse number and pulse length can be chosen.

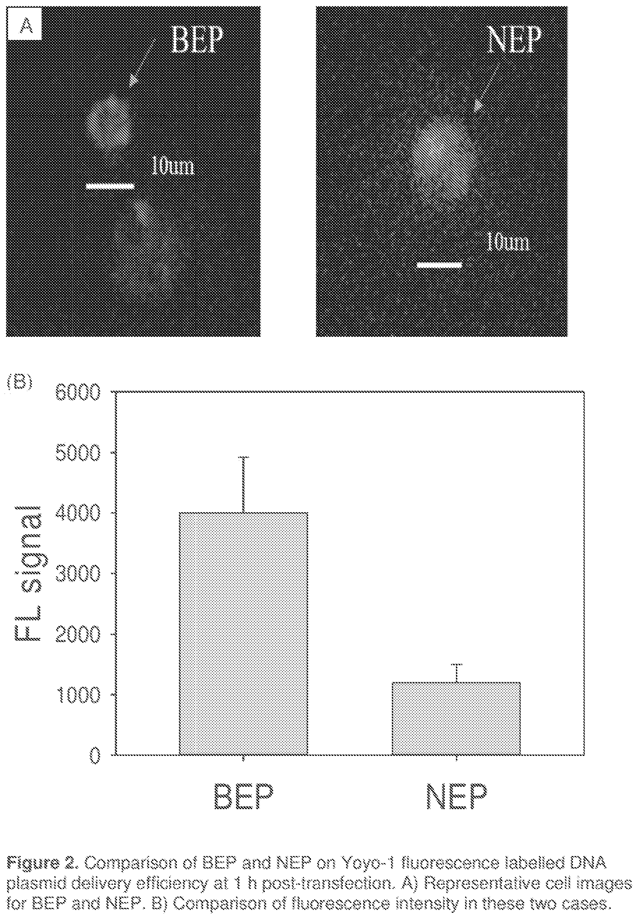

Using Yoyo-1 fluorescence dye labelled Achaete-Scute Complex Like-1 (Ascl1) DNA plasmid, **Figure 2** shows transfected cells imaged using fluorescence microscopy 1 h after transfection by either BEP or NEP under a wavelength of 488 nm. The fluorescence intensity was calculated by NIS software. Comparison of fluorescence intensity in these two groups is given as bar charts. The results show that BEP at the manufacturer recommended best conditions could deliver nearly 3 folds more plasmids than NEP at 220 volts with five 10-ms pulses to the MEF cells. However, most plasmids were still near the cell surface 1 h after BEP transfection, while the injected plasmids by NEP have already been uniformly diffused within cytoplasm at the same time. This implies that BEP based cell transfection relies mainly on electroporation-mediated endocytosis, while NEP based cell transfection is non-endocytic.

**Figure 3** compares EV numbers secreted from the same number of MEF cells (5E6 cells) transfected with the same Ascl1, Brn2 and Myt1l DNA plasmids at a weight ratio of 2/1/1 by either lipofectamine (Lipo), BEP or NEP. All EVs were collected from cell culture medium at 24 h post-transfection and the total EV number was determined by NanoSight^{™}. For BEP, the transfection voltage was 1250 v with one 30-ms pulse. For NEP, the transfection voltage was 220 with five 10 ms pulses. The concentration of plasmid used was Ascl1/Brn2/Myt1l=200/100/100 ng/µl. For lipofectamine transfection, 5 µg plasmid mixture (Ascl1/Brn2/Myt1l=2/1/1) was used according to manufacturer's instruction. The EVs were collected from cell culture medium by simply centrifugation at 1500 g for 10 mins. The results show that lipofectamine (Lipo) based cell transfection did not change the EV secretion. The EV concentration was around 2E9/ml with or without transfection. Apparently, a slow plasmid endocytosis process by nanoparticle carriers would not stimulate the transfected cells much and, consequently, there was almost no change on EV secretion. In comparison, BEP based cell transfection led to more EV secretion to ~6E9/ml. A tremendous increase of EV secretion to >1.3E11/ml was observed by NEP cell transfection with or without adding plasmids. This implies that the transfected cells were somewhat stimulated by BEP, but highly stimulated by NEP leading to very significant increase of EVs in the latter case.

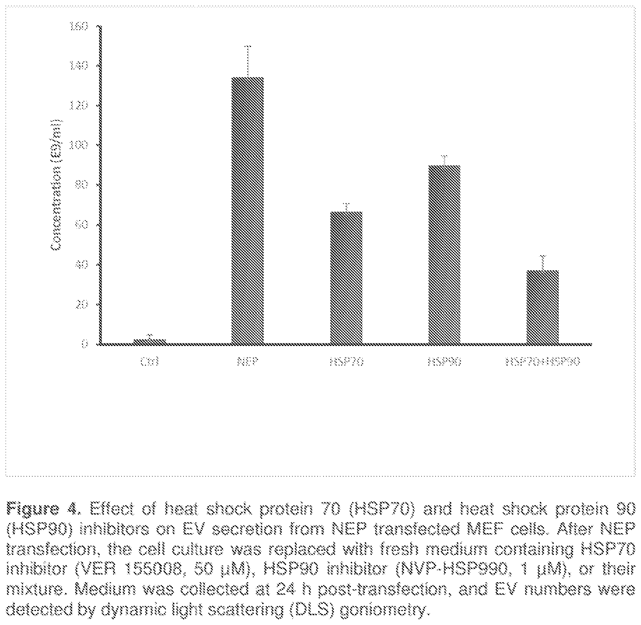

During electroporation, Joule heating caused by the imposed electric field could tentatively increase the cell temperature to cause thermal shocking to the transfected cells. It is known that thermal shocking may increase cell secretion of EVs due to chaperone mediated autophage caused by the increase of heat shock proteins in cells (8-10). Indeed, we found that NEP could substantially increase the expression of both heat shock protein 70 (HSP70) by 13.8 folds and heat shock protein 90 (HSP90) by 4.2 folds in the transfected MEF cells vs. the non-transfected MEF cells (Ctrl). When HSP inhibitors were added in cell culture medium after electroporation, EV secretion could be suppressed. **Figure 4** shows 50%, 40% and 70% decrease of EV secretion of NEP transfected MEF cells with HSP 70 inhibitor (VER 155008, , 50 µM) HSP90 inhibitor (NVP-HSP990, 1 µM), and their mixture respectively. Here, the cell culture was replaced with fresh medium containing HSP70 inhibitor (VER 155008), HSP90 inhibitor (NVP-HSP990), or their mixture right after NEP transfection. Medium was collected at 24 h post-transfection and EV numbers were detected by dynamic light scattering (DLS) goniometry. These results imply that any cell stimulation that can increase the expression of heat shock proteins would enhance EV secretion.

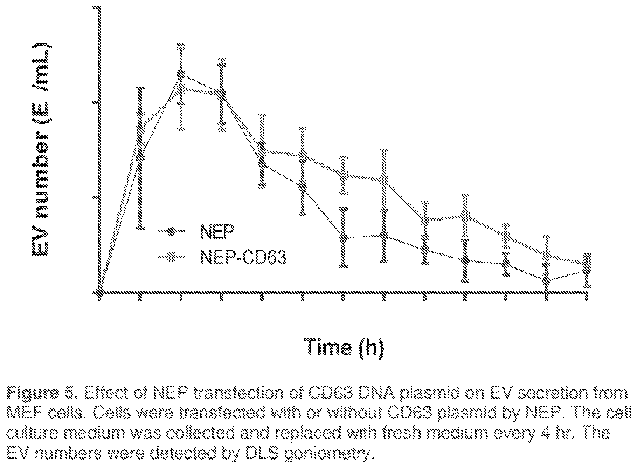

Similarly, an increase of proteins that are needed for late endosomal multi-vescular body (MVB) formation in cells may also enhance exosome secretion. **Figure 5** shows the effect of NEP transfection of CD63 DNA plasmid on EV secretion from MEF cells. Cells were transfected with or without CD63 DNA plasmid by NEP. The cell culture medium was collected and replaced with fresh medium every 4 h. The EV numbers were detected by DLS goniometry. The results show a similar EV secretion profile during the first 16 h after NEP transfection in both cases. However, more EVs were secreted between 16 to 44 h after NEP transfection with CD63 DNA plasmid. CD63 protein is essential for the reorganization of endosomal membrane into tetraspanin enriched microdomains, a precursor of exosome secretion.

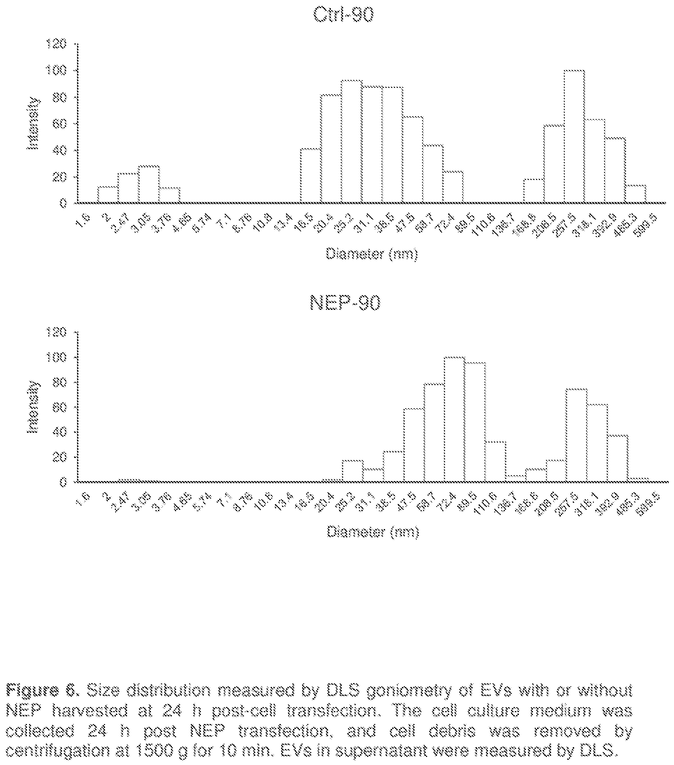

**Figure 6** shows the EV size distribution measured by DLS goniometry for MEFs (ctrl) and NEP transfected MEFs. NEP stimulation did not change the larger EV (mostly microvesicles) distribution much, but substantially increased the secretion of exosomes with sizes ranging from 40 to 110 nm.

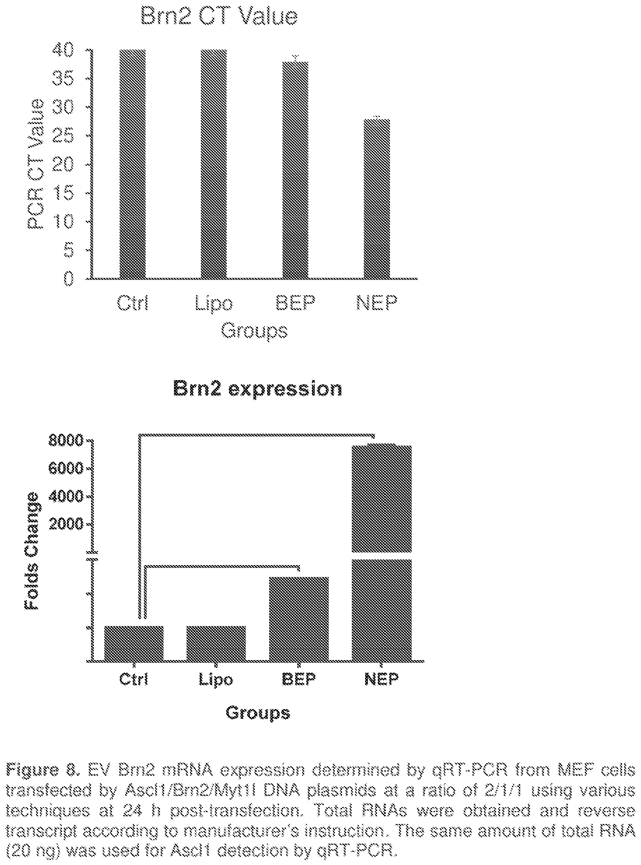

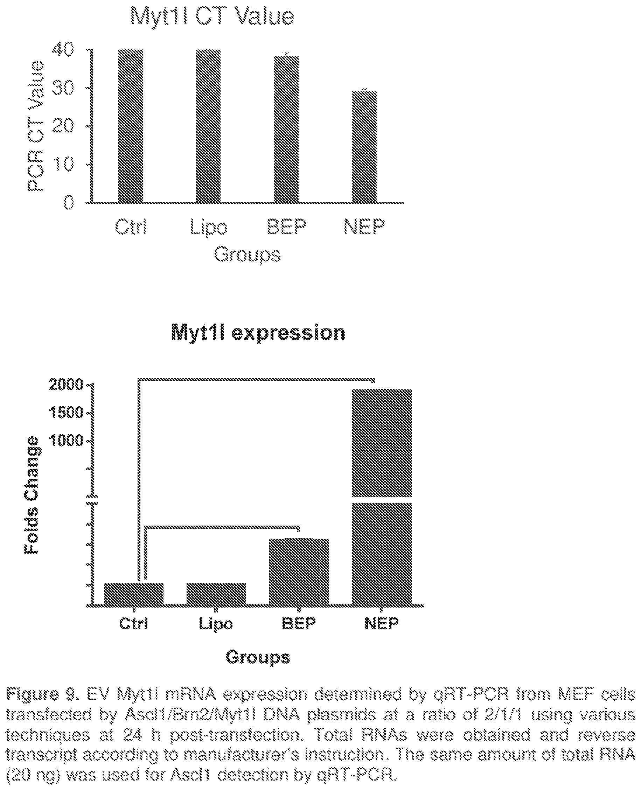

**Figures 8-9** show that the secreted EVs from NEP cell transfection of Ascl1, Brn2 and Myt1l DNA plasmids contain a large amount of corresponding Ascl1, Brn2 and Myt1l mRNAs or their fragments as determined using quantitative-Reverse Transcription Polymerase Chain Reaction (qRT-PCR). Like the EV numbers, lipofectamine (Lipo) based cell transfection did not change the mRNA expression much, while the BEP based cell transfection could increase the mRNA expression several folds. In comparison, the NEP based cell transfection resulted in thousands folds increase of target mRNAs. Here, the same amount of total RNAs were obtained and reverse transcription was conducted by qRT-PCR according to manufacturer's instruction.

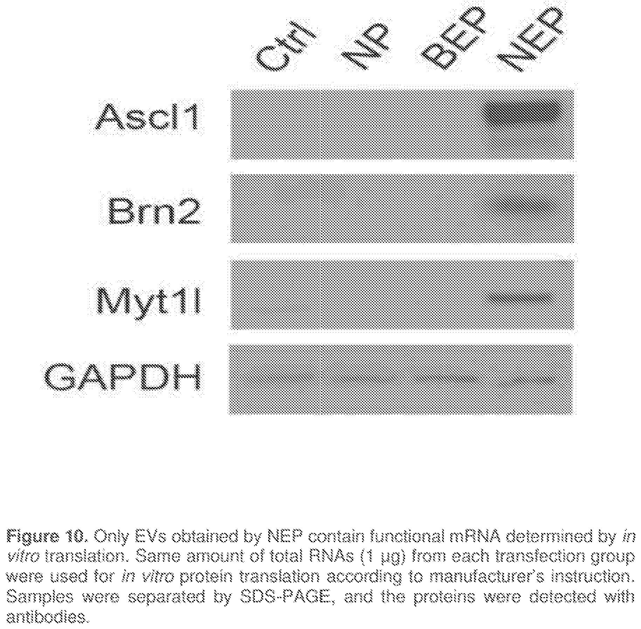

**Figure 10** shows that some of the EV mRNAs were intact and functional because they were able to translate Ascl1, Brn2 and Myt1l proteins. Here, a same amount of total RNA (1 µg) from each transfection method was applied for *in vitro* protein translation using Rabbit Reticulocyte Lysate System (Promega) according to manufacturer's instruction. Samples were separated by SDS-PAGE and the proteins were detected with various antibodies as shown in the Western blotting plot.

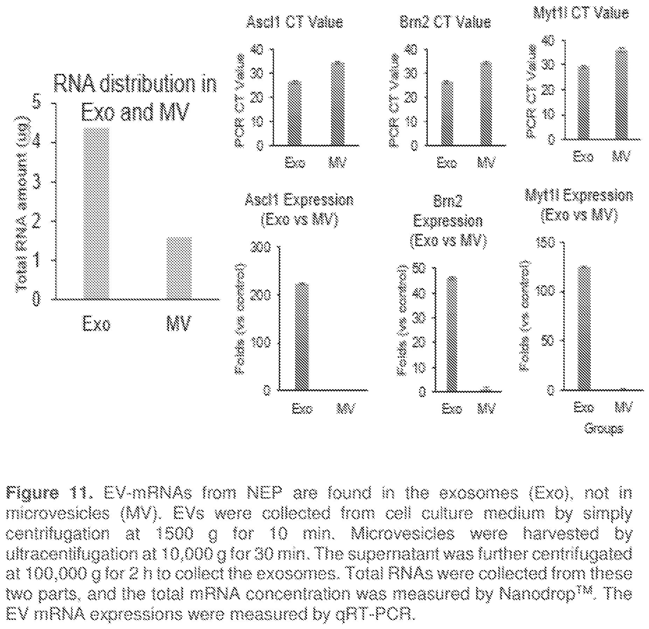

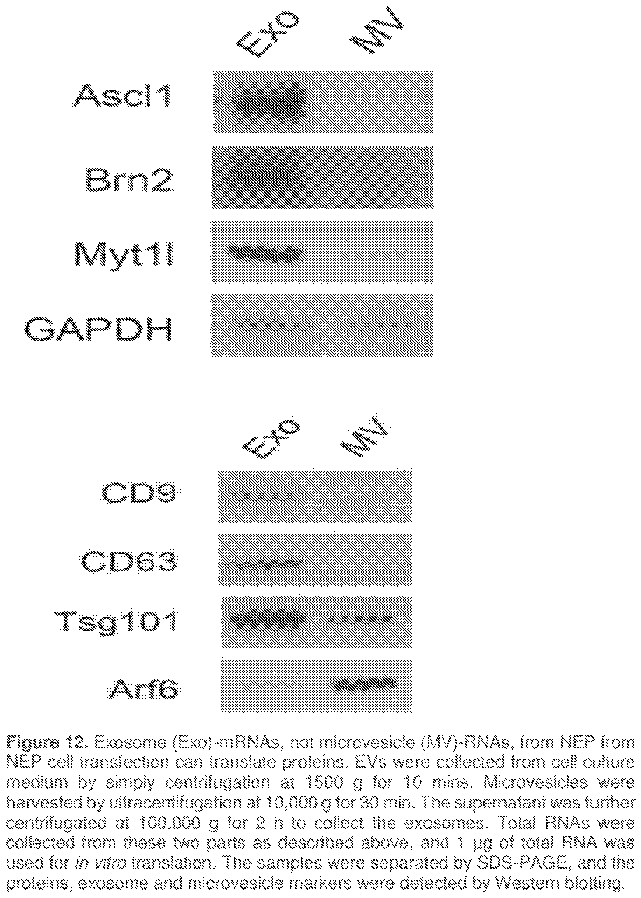

For the collected total EVs, the larger microvesicles were sorted by ultracentifugation at 10,000 g for 30 min. The supernatant was further centrifugated at 100,000 g for 2 h to collect the smaller exosomes. Total RNAs were collected from these two parts as described above. The total mRNA concentration was measured by Nanodrop^{™}, while the ABM expressions of Ascl1, Brn2 and Myt1l mRNAs were measured by qRT-PCR. **Figure 11** shows that there was more than twice RNA in exosomes than in microvesicles, but most Ascl1, Brn2 and Myt1l mRNAs were presented only in exosomes. **Figure 12** shows that the functional Ascl1, Brn2 and Myt1l mRNAs were also presented in exosomes and those exosomes carry typical exosomal protein markers, CD9, CD63 and Tsg101. In comparison, the larger microvesicles carry the typical protein marker, Arf6.

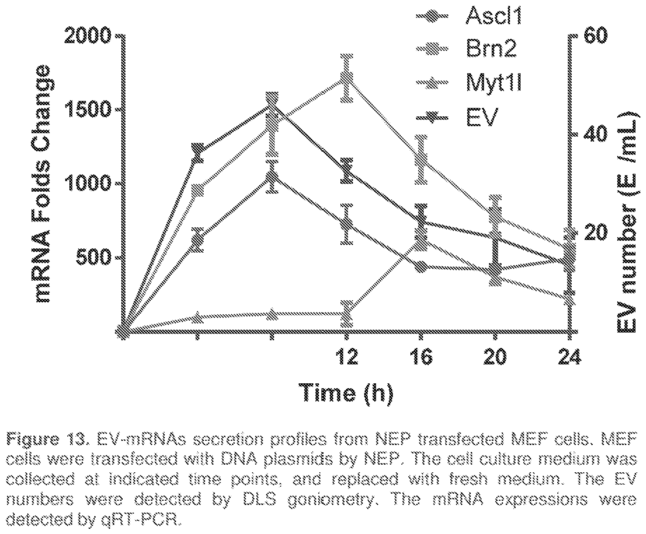

**Figure 13** shows the EV secretion and content profiles as a function of time after NEP transfection with Ascl1, Brn2 and Myt1l DNA plasmids. The Ascl1 plasmid is the smallest one (7k bp) among the three, while the Myt1l plasmid is the largest (9k bp) with the Brn2 plasmid in between (8k bp). EVs in the cell culture medium was collected at the indicated time points, and the culture medium was replaced with fresh medium. The EV numbers were detected by DLS goniometry, while the EV mRNA expressions were detected by qRT-PCR as described before. The results show a quick increase of EV secretion within 4 h post-transfection, and peaked at 8 h with continuous EV secretion for more than 24 h. EVs containing Ascl1 and Brn2 mRNAs also appeared within 4 h post-transfection with profiles matching well with that of EV secretion. EVs containing Myt1l mRNA appeared at a later time, but still within 24 h. This implies that the EV secretion time and the mRNA transcription time need to be matched in cell, which can be achieved by NEP based cell transfection.

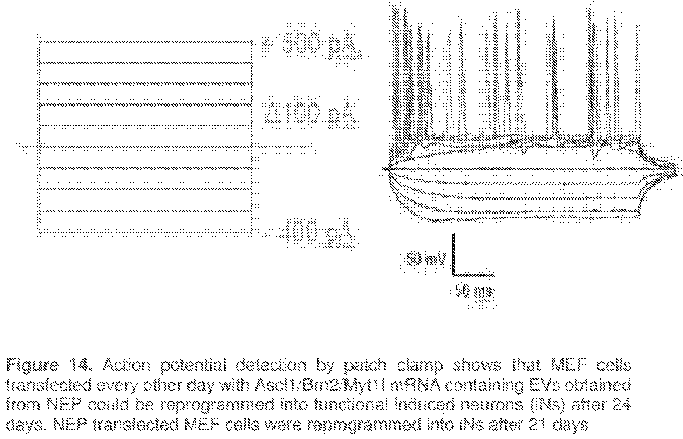

To demonstrate that NEP-produced-EVs containing endogenous mRNAs have therapeutic functions, we treated MEF cells with those EVs every other day at a total EV RNA concentration of 1 µg per 100,000 cells. After several days, the treated MEF cells started to reveal neuron-like morphology and at 24 days, the treated cells showed electrophysiological activity as demonstrated by their capacity to undergo induced action potentials as shown in **Figure 14****.** In comparison, the NEP-transfected MEF cells also showed a similar electrophysiological activity on Day 21. Cells displayed the necessary voltage-gated currents to fire action potentials. Both transient inward currents and sustained outward currents were observed in response to depolarizing voltage simulations. A typical response to a 20 pA current injection is illustrated in **Figure 14** and indicates that cells fired action potentials in response to depolarizing current.

Whole-cell patch clamp recording was used to measure excitability. Cells were continuously superfused with an extracellular bath solution containing 115 mM NaCl, 2 mM KCI, 1.5 mM MgCl₂, 3 mM CaCl₂, 10 mM HEPES, and 10 mM Glucose (pH 7.4). Glass electrodes (3-4 MΩ) were filled with a pipette solution containing 115 mM K-gluconate, 10mM N-2-Hydroxyethylpiperazine-N'-2-Ethanesulfonic Acid (HEPES), 4 mM NaCl, 0.5 mM ethylene glycol tetraacetic acid (EGTA), , 1.5 mM MgCl₂, (pH 7.3). Cells had a patch resistance of >100 MOhm after whole-cell access was gained, and series resistance was compensated 40-50%. Data were collected using an Axopatch 200B amplifier, Digidata 1322A digitizer, and Clampex 9 software (Molecular Devices, Sunnyvale, CA). For analysis of voltage-gated currents, the basal holding potential was -70 mV and cells were stepped for 400 ms in 10 mV increments from -120 mV to 80 mV. Transient inward currents, due to activity of voltage-gated sodium channels, were isolated from measuring the peak amplitude. Sustained plateau currents, reflective of voltage-gated potassium currents, were measured as the average of the last 50ms of the voltage step in the plateau phase of the current. Action potential induction was measured using current clamp. Current was held at 0 pA and then stepped in 20 pA intervals for 1 sec.

### Example 2- EV MicroRNA Content Using Different Transfection Methods.

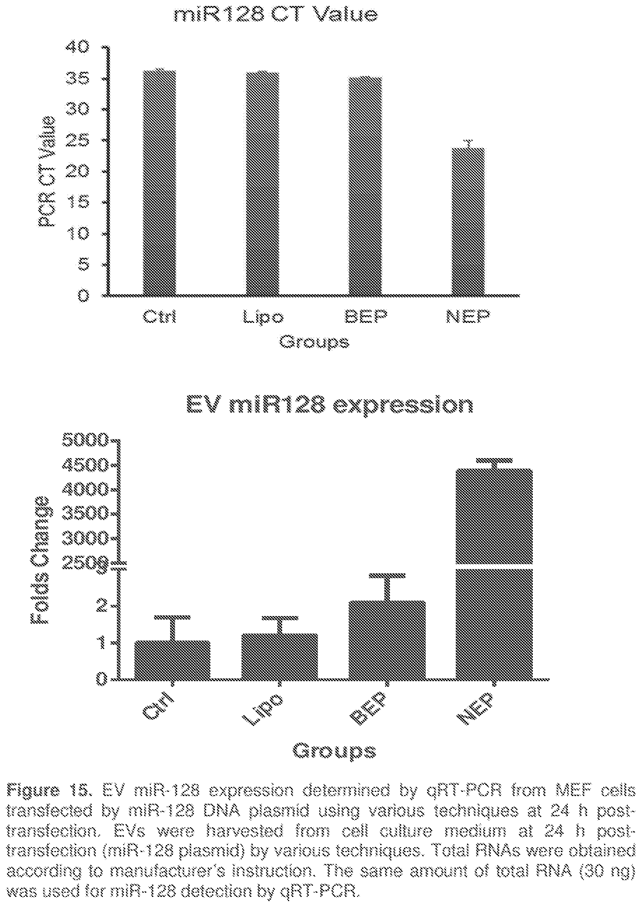

To demonstrate broader therapeutic applicability, we also transfected MEF cells with a DNA plasmid that will transcribe microRNA targets in cells. **Figure 15** shows the EV miR-128 expression for EVs harvested from cell culture medium at 24 h post-transfection (miR-128 plasmid) by various techniques. Total RNAs were obtained according to manufacturer's instruction. The same amount of total RNA (30 ng) was used for miR-128 detection by qRT-PCR using the aforementioned procedures. Again, NEP based transfection was able to produce EVs containing a large amount of miR-128 (more than 4,500 folds increase), not achievable by BEP or lipofectamine based cell transfection.

### Example 3- Comparison of EVs Containing Endogenous RNAs by NEP Transfection of DNA Plasmid to MEF Cells vs. Existing EVs Loaded with Pre-collected RNAs by BEP Post-insertion.

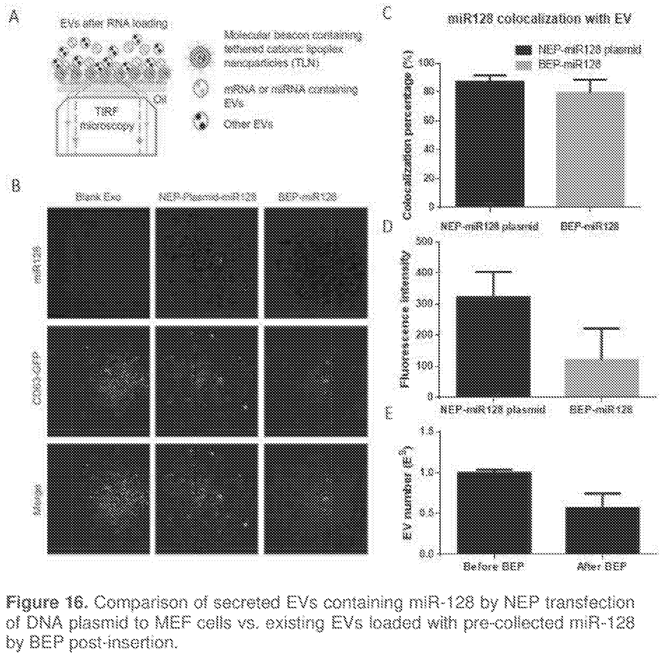

Here, we compared the efficacy of producing therapeutic EVs using our NEP based cell transfection and the BEP post-insertion approach used by several researchers. For the former, the miR-128 plasmid was co-transfected with CD63-GFP plasmid to MEF cells by NEP to generate EVs containing miR-128 according to aforementioned procedures. For the latter, blank EVs were first harvested from MEF cells transfected with CD63-GFP plasmid 24 h after NEP. In parallel, miR-128 was collected from MEF cells transfected with miR-128 plasmid 24 h post-transfection by NEP. The collected miR-128 (1 µg) was mixed with blank EVs (10E6) and electroporated by BEP (1250 volts, 30 ms) according to conditions used by other researchers. EVs from the two approaches were tested using a tethered lipoplex nanoparticle (TLN) biochip on a total internal reflection fluorescence (TIRF) microscope. **Figure 16A** shows the TLN-TIRF assay schematic (2, 11). Briefly, a molecular beacon (MB) for the RNA target is designed and encapsulated in cationic liposomal nanoparticles. These cationic lipoplex nanoparticles are tethered on a glass slide, which are able to capture negatively charged EVs by electrical static interactions to form a larger nanoscale complex. This lipoplex-EV fusion leads to mixing of RNAs and MBs within the nanoscale confinement near the biochip interface. TIRF microscopy is capable of detecting a single biomolecule and it measures signals <300 nm near the interface, which is where the tethered liposomal nanoparticles locate.

**Figure 16B** shows the representative TLN-TIRF images of the captured EVs. The green fluorescence is from EVs containing CD63-GFP, while the red fluorescence is from hybridization of miR-128 molecules and the Cy5-miR128 MBs in the captured EVs. It is clear that our NEP approach is able to produce more EVs containing higher copies of miR-128 than the BEP post-insertion approach. **Figures 16C-E** show a quantitative comparison of those two approaches. Although both approaches are able to produce EVs containing miR-128 (~80% of total captured EVs), the EV miR-128 concentration in EVs (~3 times MB fluorescence intensity) is much higher in NEP based direct cell transfection than in BEP based microRNA post-insertion. Furthermore, BEP post-insertion tends to break nearly half of the blank EVs leading to a very low yield of therapeutic EVs.

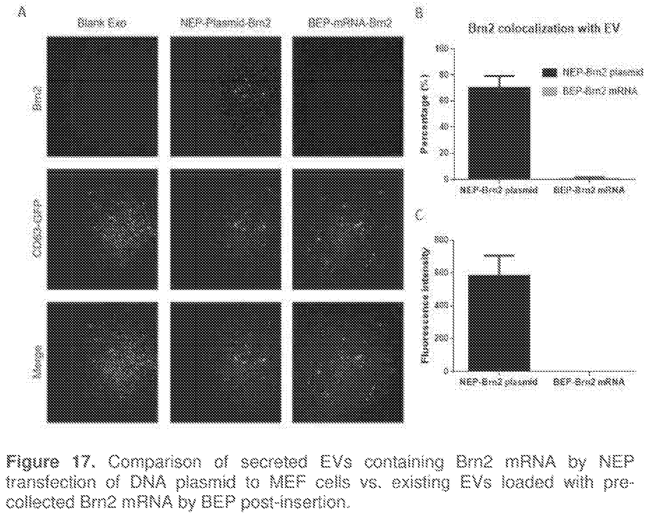

A similar comparison was also carried out for a much larger RNA, Brn2 mRNA (6272 bases for Brn2 mRNA vs. 21 bases for miR-128) using the same approach as for miR-128. **Figure 17** shows that our NEP approach could produce >70% EVs containing Brn2 mRNA, while only very few existing EVs could be loaded with the same mRNA by BEP post-insertion approach. The concentration of Brn2 mRNA in NEP produced EVs is high, while that in BEP post-insertion is very poor.

### Example 4- Improvement of Multiple mRNAs Co-localized in the Same Secreted EVs by Sequential NEP Transfection of DNA Plasmids to MEF Cells.

**Figure 13** implies that different mRNA targets could be transcribed at different times and rates in the transfected cells, even though multiple DNA plasmids were delivered to the cells at the same time, due to the size difference of plasmids or other reasons. This may lead to individual EVs containing only one or few mRNA targets. For better therapeutic efficacy, it would be valuable if more or all mRNA targets can be encapsulated in the same secreted EVs. By sequentially delivering each DNA plasmid into MEF cells using NEP based on its transcription time, **Figure 18** shows that we could substantially increase the secreted EVs containing all three mRNAs, Ascl1, Brn2 and Myt1l (>50% vs. <25%), needed for iN reprogramming. For NEP transfection, Ascl1, Brn2 and Myt1l plasmids were transfected at the same time as described before. For sequential-NEP, the Myt1l plasmid was transfected first, Brn2 plasmid was transfected 4 h later, while Ascl1 plasmid was transfected 4 h after Brn2 transfection. At 24 h post Myt1l transfection, culture medium was collected for TLN assay. Equal amount of FAM-Ascl1, Cy3-Brn2, and Cy5-Myt1l MBs were encapsulated in tethered lipoplex nanoparticles for EV-mRNA detection. In the figure, the yellow arrow means EVs containing all 3 mRNAs, the blue arrow means EVs containing 2 mRNAs, while the pink arrow means EVs containing only 1 mRNA.

While the invention has been explained in relation to its preferred embodiments, it is to be understood that various modifications thereof will become apparent to those skilled in the art upon reading the specification. Therefore, it is to be understood that the invention disclosed herein is intended to cover such modifications as fall within the scope of the appended claims.

## Claims

1. A method of producing a large number of therapeutic extracellular vesicles (EVs) comprising high copies of a biomolecule comprising the steps of:
laying donor cells on a surface of a chip, the surface having a three-dimensional (3D) nanochannel electroporation (NEP) biochip formed thereon;
adding a plasmid, other transfection vector, or their combinations to a buffer on the chip;
applying a pulsed electric field across the donor cells laid on top of the chip surface and plasmid/vector buffer solution below the chip surface, resulting in strongly stimulating the donor cells and delivering the plasmid/vector into the donor cells nonendocytically; and
collecting EVs secreted by the transfected donor cells.

2. The method of claim 1, wherein the diameter of nanochannels of the 3D NEP biochip is between 50-900 nm.

3. The method of claim 1 or 2, wherein the plasmid or vector transcribes messenger RNA (mRNA), microRNA (miRNA), short hairpin RNA (shRNA), or another RNA, and, optionally, leads to translation of a protein or other biomolecule in the transfected donor cells.

4. The method of any one of the preceding claims, wherein EVs secreted by the transfected donor cells comprise the transcribed messenger RNA (mRNA), microRNA (miRNA), short hairpin RNA (shRNA), or another RNA, and, optionally, the translated proteins or other biomolecule.

5. The method of any one of the preceding claims, further comprising increasing the expression of heat shock proteins and other proteins that promote vesicle formation and exocytosis in the transfected donor cells by thermal shock treatment of the donor cells, or addition of heat shock proteins.

6. The method of any one of the preceding claims, further comprising increasing the expression of proteins that promote exosome formation in the transfected donor cells by co-transfection of CD63, CD9, and other DNA plasmid.

7. The method of any one of the preceding claims, wherein multiple DNA plasmids or other vectors are delivered to the transfected donor cells sequentially to promote co-localization of RNA targets or protein targets and EV secretion.

8. The method of any one of the preceding claims, wherein exogenous biomolecules such as a DNA plasmid, other transfection vectors, RNAs, proteins/peptides, or small molecule drugs are encapsulated within vesicles in the donor cells and secreted out as therapeutic EVs by sequential transfection of the donor cells by NEP.

9. The method of claim 8, wherein, in addition to NEP, one or more cell transfection methods are used to provide strong stimulation to the donor cells to facilitate EV secretion and non-endocytic plasmid/vector delivery for fast RNA transcription and protein translation to produce therapeutic EVs with similar efficacy.

10. The method of claim 9, wherein the other cell transfection methods include gene gun, microinjection, or nanoinjection.

11. The method of claim 9 or 10, wherein the cell transfection method comprises tethering the plasmid and/or other vector on nano- or micron-sized gold or other solid particles, and those particles are injected into the donor cells under a pneumatic force using a gene gun to cause strong stimulation of the donor cells and non-endocytic plasmids/vector delivery.

12. The method of any one of claims 9 to 11, wherein the cell transfection method comprises tethering the plasmid and/or other vector on a nano- or micron-sized tip array, and the donor cells are pultruded by those tips to cause strong stimulation of the donor cells and non-endocytic plasmids/vector delivery into the donor cells.

13. The method of claim 1, wherein the plasmid or transfection vector is a DNA plasmid or DNA transfection vector.

14. The method of claim 13, wherein the EVs secreted by the transfected cells comprise therapeutic RNA.

## Patentansprüche

1. Verfahren zum Herstellen einer großen Anzahl von therapeutischen extrazellulären Vesikeln (EVs), umfassend hohe Kopienzahlen eines Biomoleküls, umfassend die Schritte:
Aufbringen von Spenderzellen auf eine Oberfläche eines Chips, wobei die Oberfläche einen darauf gebildeten dreidimensionalen (3D) Nanokanal-Elektroporationsbiochip (NEP-Biochip) aufweist;
Zugeben eines Plasmids, eines anderen Transfektionsvektors oder deren Kombinationen zu einem Puffer auf dem Chip;
Anlegen eines gepulsten elektrischen Feldes über die auf der Chip-Oberfläche liegenden Spenderzellen und die Plasmid-/Vektor-Pufferlösung unterhalb der Chip-Oberfläche hinweg, was ein starkes Stimulieren der Spenderzellen und eine nicht-endozytotische Abgabe des Plasmids/Vektors in die Spenderzellen zur Folge hat; und
Sammeln von EVs, die durch die transfizierten Spenderzellen sezerniert werden.

2. Verfahren nach Anspruch 1, wobei der Durchmesser von Nanokanälen des 3D-NEP-Biochips zwischen 50-900 nm liegt.

3. Verfahren nach Anspruch 1 oder 2, wobei das Plasmid oder der Vektor Messenger-RNA (mRNA), microRNA (miRNA), Short-Hairpin-RNA (shRNA) oder eine andere RNA transkribiert und optional zu einer Translation eines Proteins oder anderen Biomoleküls in den transfizierten Spenderzellen führt.

4. Verfahren nach einem der vorstehenden Ansprüche, wobei EVs, die durch die transfizierten Spenderzellen sezerniert werden, die transkribierte Messenger-RNA (mRNA), microRNA (miRNA), Short-Hairpin-RNA (shRNA) oder eine andere RNA und optional die translatierten Proteine oder ein anderes Biomolekül umfassen.

5. Verfahren nach einem der vorstehenden Ansprüche, ferner umfassend ein Erhöhen der Expression von Hitzeschockproteinen und anderen Proteinen, die die Vesikelbildung und Exozytose fördern, in den transfizierten Spenderzellen durch thermische Schockbehandlung der Spenderzellen oder Zugabe von Hitzeschockproteinen.

6. Verfahren nach einem der vorstehenden Ansprüche, ferner umfassend das Erhöhen der Expression von Proteinen, die die Exosomenbildung in den transfizierten Spenderzellen fördern, durch Cotransfektion von CD63, CD9 und einem anderen DNA-Plasmid.

7. Verfahren nach einem der vorstehenden Ansprüche, wobei mehrere DNA-Plasmide oder andere Vektoren zu den transfizierten Spenderzellen sequenziell abgegeben werden, um eine Kolokalisation von RNA-Zielen oder Proteinzielen und die EV-Sekretion zu fördern.

8. Verfahren nach einem der vorstehenden Ansprüche, wobei exogene Biomoleküle wie ein DNA-Plasmid, andere Transfektionsvektoren, RNAs, Proteine/Peptide oder niedermolekulare Arzneistoffe innerhalb von Vesikeln in den Spenderzellen eingekapselt und durch sequentielle Transfektion der Spenderzellen durch NEP als therapeutische EVs sezerniert werden.

9. Verfahren nach Anspruch 8, wobei zusätzlich zu NEP ein oder mehrere Zelltransfektionsverfahren verwendet werden, um den Spenderzellen eine starke Stimulation bereitzustellen, um die EV-Sekretion und die nicht-endozytische Plasmid-/Vektorabgabe für eine schnelle RNA-Transkription und Proteintranslation zu erleichtern, um therapeutische EVs mit ähnlicher Wirksamkeit herzustellen.

10. Verfahren nach Anspruch 9, wobei die anderen Zelltransfektionsverfahren Genkanone, Mikroinjektion oder Nanoinjektion einschließen.

11. Verfahren nach Anspruch 9 oder 10, wobei das Zelltransfektionsverfahren ein Anbinden des Plasmids und/oder anderen Vektors an nano- oder mikrometergroße Gold- oder andere Feststoffpartikel umfasst, und diese Partikel unter einer pneumatischen Kraft unter Verwendung einer Genkanone in die Spenderzellen injiziert werden, um eine starke Stimulation der Spenderzellen und eine nicht-endozytotische Plasmid-/Vektorabgabe zu bewirken.

12. Verfahren nach einem der Ansprüche 9 bis 11, wobei das Zelltransfektionsverfahren das Anbinden des Plasmids und/oder eines anderen Vektors an ein nano- oder mikrometergroßes Spitzenarray umfasst, und die Spenderzellen durch diese Spitzen pultrudiert werden, um eine starke Stimulation der Spenderzellen und eine nicht-endozytotische Plasmid-/Vektorabgabe in die Spenderzellen zu bewirken.

13. Verfahren nach Anspruch 1, wobei das Plasmid oder der Transfektionsvektor ein DNA-Plasmid oder DNA-Transfektionsvektor ist.

14. Verfahren nach Anspruch 13, wobei die EVs, die durch die transfizierten Zellen sezerniert werden, therapeutische RNA umfassen.

## Revendications

1. Procédé de production d'un grand nombre de vésicules extracellulaires thérapeutiques (VE) comprenant des copies élevées d'une biomolécule comprenant les étapes consistant à :
poser des cellules de donneurs sur une surface d'une puce, la surface ayant une biopuce tridimensionnelle (3D) d'électroporation à nanocanaux (NEP) formée sur celle-ci ;
ajouter un plasmide, un autre vecteur de transfection, ou leurs combinaisons à un tampon sur la puce ;
l'application d'un champ électrique pulsé à travers les cellules donneuses posées sur la surface de puce et une solution tampon de plasmide/vecteur sous la surface de puce, ce qui entraîne une forte stimulation des cellules donneuses et l'administration du plasmide/vecteur dans les cellules donneuses de manière non endocytique ; et
la collecte des VE sécrétées par les cellules donneuses transfectées.

2. Procédé selon la revendication 1, dans lequel le diamètre des nanocanaux de la biopuce NEP 3D est compris entre 50 et 900 nm.

3. Procédé selon la revendication 1 ou 2, dans lequel le plasmide ou vecteur transcrit l'ARN messager (ARNm), le microARN (miARN), l'ARN en épingle à cheveux court (ARNsh), ou un autre ARN, et, éventuellement, conduit à la traduction d'une protéine ou d'une autre biomolécule dans les cellules donneuses transfectées.

4. Procédé selon l'une quelconque des revendications précédentes, dans lequel les EV sécrétées par les cellules donneuses transfectées comprennent l'ARN messager transcrit (ARNm), le microARN (miARN), l'ARN en épingle à cheveux courte (shARN), ou un autre ARN, et, éventuellement, les protéines traduites ou une autre biomolécule.

5. Procédé selon l'une quelconque des revendications précédentes, comprenant en outre l'augmentation de l'expression de protéines de choc thermique et d'autres protéines qui favorisent la formation de vésicules et l'exocytose dans les cellules donneuses transfectées par un traitement par choc thermique des cellules donneuses, ou par l'ajout de protéines de choc thermique.

6. Procédé selon l'une quelconque des revendications précédentes, comprenant en outre l'augmentation de l'expression de protéines qui favorisent la formation d'exosomes dans les cellules donneuses transfectées par co-transfection de CD63, CD9, et d'autres plasmides d'ADN.

7. Procédé selon l'une quelconque des revendications précédentes, dans lequel de multiples plasmides d'ADN ou autres vecteurs sont délivrés aux cellules donneuses transfectées séquentiellement pour favoriser la colocalisation de cibles d'ARN ou de cibles protéiques et la sécrétion d'EV.

8. Procédé selon l'une quelconque des revendications précédentes, dans lequel des biomolécules exogènes telles qu'un plasmide d'ADN, d'autres vecteurs de transfection, des ARN, des protéines/peptides ou des médicaments à petites molécules sont encapsulées dans des vésicules dans les cellules donneuses et sécrétées en tant qu'EV thérapeutiques par transfection séquentielle des cellules donneuses par NEP.

9. Procédé selon la revendication 8, dans lequel, en plus de la NEP, un ou plusieurs procédés de transfection cellulaire sont utilisés pour fournir une forte stimulation aux cellules donneuses afin de faciliter la sécrétion d'EV et l'administration de plasmides/vecteurs non endocytaires pour une transcription rapide de l'ARN et une traduction des protéines afin de produire des VE thérapeutiques avec une efficacité similaire.

10. Procédé selon la revendication 9, dans lequel les autres procédés de transfection cellulaire comportent un pistolet à gènes, une micro-injection ou une nano-injection.

11. Procédé selon la revendication 9 ou 10, dans lequel le procédé de transfection cellulaire comprend l'attache du plasmide et/ou autre vecteur sur de l'or ou d'autres particules solides de taille nanométrique ou micrométrique, et ces particules sont injectées dans les cellules donneuses sous une force pneumatique à l'aide d'un pistolet à gènes pour provoquer une forte stimulation des cellules donneuses et du plasma non endocytaire ids/livraison vectorielle.

12. Procédé selon l'une quelconque des revendications 9 à 11, dans lequel le procédé de transfection cellulaire comprend la fixation du plasmide et/ou autre vecteur sur un réseau de pointes de taille nanométrique ou micrométrique, et les cellules donneuses sont pultrudées par ces pointes pour provoquer une forte stimulation des cellules donneuses et une délivrance de plasmides non endocytaires/vecteurs dans les cellules donneuses.

13. Procédé selon la revendication 1, dans lequel le plasmide ou vecteur de transfection est un plasmide d'ADN ou un vecteur de transfection d'ADN.

14. Procédé selon la revendication 13, dans lequel les VE sécrétées par les cellules transfectées comprennent de l'ARN thérapeutique.
